# FASCICULE DE BREVET EUROPEEN

(11) **EP 0 769 499 B1**
(45) Date de publication et mention de la délivrance du brevet: **10.01.2001**
(21) Numéro de dépôt: 96402100.0
(22) Date de dépôt: 02.10.1996
(51) Int. Cl.: C07H 15/04, C11D 1/66, A61K 7/06

(54) **(Oxo-1 alkyl) amino-2, déoxy-2 glucopyranoside de dihydroxy-2,3 propyl, leur procédé de préparation et leurs utilisations**
(Oxo-1-Alkyl)amino-2-desoxy-2-glucopyranosid von Dihydroxy-2,3-propyl, Verfahren zur Herstellung und ihre Verwendungen
1-(2,3-Dihydroxypropyl)-2-deoxy-2-(alkyloxyamino)glucopyranoside their preparation and their uses

(30) Priorité: 18.10.1995 FR 9512215
(43) Date de publication de la demande: 23.04.1997
(73) Titulaire: CECA S.A., 92800 Puteaux (FR); Atofina, 92800 Puteaux (FR)
(72) Inventeur: Petit, Serge, 74540 Cusy (FR); Bernard, Daniel, 92400 Courbevoie (FR); Caupin, Henri-Jean, 78000 Versailles (FR)

(56) Documents cités:
- EP-A- 0 569 682

## Description

La présente invention a trait au domaine des agents tensio-actifs à caractère neutre. Elle concerne plus particulièrement les (oxo-1 alkyl) amino-2, déoxy-2 glucopyranoside de dihydroxy-2,3 propyl, leur procédé de préparation, les compositions renfermant lesdits composés ainsi que leurs utilisations.

Dans le domaine des agents tensio-actifs, on recherche de manière permanente des composés dépourvus de caractère toxique et irritant, notamment vis-à-vis de la peau et des muqueuses de l'utilisateur avec lesquelles ils entrent en contact, de tels composés devant, en outre, préserver l'environnement.

Les agents tensio-actifs trouvent leurs usages dans des secteurs aussi variés que la cosmétologie, les soins capillaires et la détersion en milieu industriel. Ils sont généralement mis en oeuvre sous forme de compositions qui, outre leur fonction principale, doivent désormais présenter un nombre toujours croissant de propriétés spécifiques.

Un shampooing, par exemple, n'est plus seulement recherché pour ses propriétés détergentes, mais aussi pour son activité anti-pelliculaire, ses capacités à conditionner le cheveu, prévenir les démangeaisons, conférer une sensation agréable au toucher.

Pour atteindre cet objectif, une première alternative consiste à associer un ou plusieurs agents tensio-actifs neutres avec des actifs présentant les propriétés requises.

On a ainsi proposé des agents tensio-actifs neutres, généralement polyoxyéthylénés (HEFFORD B., Surfactant Surveillance, S.P.C., pp. 35-36, Nov. 1991) et des polyalkylglucosides dérivés du glucose (EP-A-569 682; SIRACUSA P.A., Happi, pp. 100-108, avril 1992; THIEM J. et BOCKER T., Special Publication of the Royal Society of Chemistry, vol. 107, pp. 123-147, 1992; SCHULZ P., Chimica Oggi, pp. 33-38, août-septembre 1992; BALZER D., Tenside, Surf. Det. vol. 28 n° 6, pp. 419-426, 1991; SALKA B., Cosmetics & Toiletries, vol. 108, pp. 89-94, mars 1993.

Les agents tensio-actifs précités possèdent en commun un bon pouvoir détergent et une faible irritabilité des yeux, les polyalkylglucosides étant, en outre, susceptibles d'être biodégradés.

Ils ont cependant comme principal inconvénient d'être difficiles à formuler à cause de leur pouvoir moussant limité et/ou du nombre élevé d'actifs dans la composition.

Ainsi, de tels agents ne peuvent être utilisés qu'en faible quantité car il est nécessaire de leur adjoindre une base lavante et moussante constituée par un tensio-actif anionique. Le caractère irritant de ce dernier doit être tempéré par l'ajout d'un troisième agent tensio-actif amphotère.

En outre, les composés polyoxyéthylénés étant susceptibles de contenir du 1,4 dioxane suspecté d'avoir une action cancérigène, II est vraisemblable que leur taux d'incorporation devrait diminuer dans le futur.

Les polyalkylglucosides, quant à eux, sont obtenus sous forme de mélanges résultant des phénomènes d'isomérisation et d'oligomérisation inter- et/ou intramoléculaires.

Il s'ensuit que la formulation de compositions renfermant de tels agents tensio-actifs s'avère délicate.

Une seconde alternative consiste à limiter le nombre des actifs par l'utilisation d'agents tensio-actifs possédant des propriétés spécifiques.

De tels agents ont notamment été décrits par EMMERLING (Polym. Bull., vol. 6, pp. 305-308, 1982) et INOUYE et coll. (J. Am. Chem. Soc., vol. 78, pp. 2825-2832, 1956). Il s'agit de composés du type alkyl glucosamine, notamment N-acyl glucosamine, présentant des propriétés spécifiques telles que la protection contre les radiations (DE 2708667 et US 4,323,561), le diagnostic et le transport d'actifs via la formation de vésicules (WO 91/04013 et WO 88/06883), le traitement des allergies, de l'acné ou des dermatoses (EP-A-209 770), une activité immuno-stimulante (VALLANI et coll., ARZNEIM FORSCH, vol. 39 n° 10, pp. 1190-1195, 1989), une stimulation de la pousse du cheveu (EP-A-348 184) et une activité anti-microbienne (J. Am. Oil Chemists' Soc., vol. 70 (1), pp. 17-22, 1993 et JP 03112905).

L'inconvénient majeur des agents précités est leur solubilité réduite dans l'eau qui, par voie de conséquence, limite leur emploi. On ne peut espérer remédier à cet inconvénient en les transformant en chlorure (J. A. O. C. S., vol. 70(1), pp. 17-22, 1993 et JP61-57321) ou en bromhydrate car, alors, on introduit le caractère irritant propre à la plupart des agents tensio-actifs cationiques.

Il a maintenant été trouvé de nouveaux agents tensio-actifs neutres caractérisés en ce qu'ils répondent à la formule : 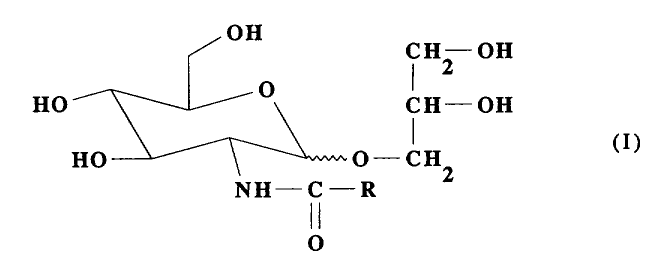 dans laquelle R représente un groupement alkyle, linéaire ou ramifié, saturé ou insaturé, renfermant de 5 à 21 atomes de carbone.

L'invention concerne tout particulièrement les composés de formule (I) dont le radical R contient de 7 à 17 atomes de carbone et mieux encore de 9 à 13.

Dans la présente description, les composés de formule (I) sont dénommés (oxo-1 alkyl) amino-2, déoxy-2 glucopyranoside de dihydroxy-2,3 propyl.

L'invention concerne aussi un procédé de préparation des composés de formule (I), ce procédé étant caractérisé en ce qu'il consiste à :
a) former une N-acyl glucosamine à partir de chlorhydrate de glucosamine et de chlorure d'acide en C₆-C₂₂, et
b) faire réagir le composé de l'étape a) avec le glycérol en présence d'un catalyseur acide,

Le chlorhydrate de glucosamine du procédé selon l'invention est généralement obtenu selon les méthodes classiques d'hydrolyse acide de la chitine.

Le chlorure d'acide du procédé selon l'invention est choisi parmi les chlorures d'acide en C₆-C₂₂ et de préférence C₈-C₁₈, ayant un radical alkyle linéaire ou ramifié, saturé ou insaturé, à l'exception du chlorure d'acide undécylénique. Ce chlorure peut être préparé notamment par réaction de l'acide correspondant avec du chlorure de thionyle à une température de 60-70°C et distillation sous pression réduite du chlorure d'acide obtenu.

Le catalyseur acide du procédé selon l'invention est choisi parmi le groupe constitué par les acides en général, par exemple l'acide chlorhydrique ou sulfurique, un acide alkyl sulfurique tel que l'acide décyl ou lauryl sulfurique, un acide sulfonique tel que l'acide benzène sulfonique, paratoluène sulfonique ou camphrosulfonique, un acide alkyl sulfonique tel que l'acide méthane-sulfonique, décyl sulfonique, lauryl sulfonique ou sulfosuccinique, un sulfosuccinate d'alkyle tel que le sulfosuccinate de décyl ou de lauryl, un acide perhalohydrique tel que l'acide perchlorique, l'acide hypophosphoreux ou les mélanges de ces acides, les résines sous forme H⁺ telles que les résines sulfoniques et les argiles acides. De préférence, on utilise l'acide sulfurique, l'acide méthane-sulfonique, l'acide succinique ou un sulfosuccinate d'alkyle, l'acide hypophosphoreux et les mélanges de ces acides.

L'étape a) du procédé selon l'invention consiste à faire réagir le chlorure d'acide et le chlorhydrate de glucosamine dans un rapport molaire généralement compris entre 0,7 et 1,3 et de préférence entre 0,9 à 1,1.

En général, on solubilise le chlorhydrate de glucosamine dans de la soude aqueuse de concentration comprise entre 6 et 60 g/litre et de préférence 2,5 et 17,5 g/litre. La concentration de chlorhydrate de glucosamine dans la solution est généralement comprise entre 0,2 et 1 équivalent en mole/litre et de préférence 0,285 et 0,5 équivalent en mole/litre.

A la solution de chlorhydrate de glucosamine ainsi préparée, on ajoute simultanément le chlorure d'acide et une solution de soude de normalité comprise entre 1 et 10 et de préférence 2 à 6.

L'ajout de chlorure d'acide est généralement effectué à un débit compris entre 5 et 500 ml/heure et de préférence 10 et 100 ml/heure.

Le débit de la solution de soude est adapté de telle sorte que le pH du mélange réactionnel soit compris entre 6 et 11 et de préférence 8 et 10,5.

La température du mélange réactionnel est généralement comprise entre 0 et 80°C et de préférence 10 et 50°C.

Le milieu réactionnel est avantageusement maintenu sous une agitation comprise entre 50 et 800 tours/min. et de préférence 100 et 400 tours/min. notamment à l'aide d'un dispositif à double pâle.

Après l'addition du chlorure d'acide, on laisse la réaction se poursuivre dans les conditions de température et d'agitation précitées pendant un temps pouvant varier de 0,25 à 24 heures et de préférence de 0,5 à 6 heures.

A l'issue de la réaction, on ajuste le pH du milieu réactionnel à une valeur comprise entre 4 et 8 et de préférence 5 et 7 par addition d'un acide afin d'obtenir la formation d'un précipité. Il va de soi que l'on peut utiliser tout type d'acide connu de l'homme du métier. De manière avantageuse, on utilise l'acide sulfurique, l'acide méthane-sulfonique, l'acide succinique, un sulfosuccinate d'alkyle, l'acide hypophosphoreux et les mélanges de ces acides.

Le précipité du mélange réactionnel précité peut être récupéré de deux manières.

Selon une première variante, le précipité est récupéré par essorage et lavage à l'eau en continu.

Selon une deuxième variante, le précipité est récupéré par filtration du milieu réactionnel, essoré, lavé 1 à 4 fois, et de préférence 2 à 3 fois, avec 0,1 à 2 litres et de préférence 0,4 à 1 litre d'eau et essoré à nouveau.

Le précipité obtenu à l'issue de l'étape a) est constitué de N-acyl glucosamine.

Dans l'étape b) du procédé selon l'invention, on fait réagir le précipité de N-acyl glucosamine précité avec du glycérol en présence d'un catalyseur acide.

Le glycérol est généralement mis en oeuvre dans le procédé de sorte que le rapport du nombre d'équivalents en mole de glycérol au nombre d'équivalents en mole de N-acyl glucosamine soit compris entre 1 et 50 et de préférence 5 et 30.

En général, lorsque le catalyseur acide tel que défini précédemment est un acide, le rapport du nombre d'équivalents en mole de l'acide au nombre d'équivalents en mole de N-acyl glucosamine est compris entre 10⁻³ et 1 et de préférence 10⁻² et 10⁻¹. Lorsque ce catalyseur est une résine ou une argile, la quantité utilisée, exprimée en équivalent pondéral calculé sur la base de 1 équivalent en mole de N-acyl glucosamine, est comprise entre 0,05 et 6.

Lorsqu'un un effet déshydratant est recherché, on peut éventuellement ajouter un agent de déshydratation classique tel qu'un tamis moléculaire ou une zéolite dans le milieu réactionnel.

La réaction est généralement effectuée sous une pression comprise entre 0,013 et 101,32 kPa, de préférence 0,013 et 39,99 kPa, à une température comprise entre 25 et 200°C, de préférence 80 et 105°C, et pendant une durée pouvant varier de 0,25 à 24 heures, de préférence 1 à 12 heures.

A l'issue de la réaction, le catalyseur est éliminé par filtration. Lorsque ce dernier est un acide, il est préalablement transformé en sel de l'acide correspondant, par exemple par l'addition d'une solution d'hydrogénocarbonate de sodium.

Dans le but de faciliter l'étape de filtration, on peut éventuellement utiliser un solvant choisi parmi les étheroxydes, par exemple le tétrahydrofurane, l'éther diméthylique de l'éthylène glycol, les hydrocarbures halogénés, par exemple le dichloro-méthane, le dichloroéthane et le chloroforme, les esters, par exemple l'acétate d'éthyle, l'acétate de propyle et l'acétate de butyle, les alcools, par exemple le méthanol, l'éthanol et le propanol, les solvants du type amide, par exemple le N-méthyl formamide et le N,N-diméthyl formamide, et les mélanges de ces solvants. De préférence, on utilise un alcool.

Le milieu réactionnel ainsi obtenu est une solution de (oxo-1 alkyl) amino-2, déoxy-2 glucopyranoside de dihydroxy-2,3 propyl dans le glycérol qui se présente généralement sous la forme d'un mélange des anomères α et β en position 1, la proportion de l'anomère α relativement à l'anomère β étant préférentiellement compris entre 80/20 et 90/10. Les anomères α et β peuvent éventuellement être séparés par exemple par chromatographie sur colonne de silice.

Le (oxo-1 alkyl) amino-2, déoxy-2 glucopyranoside de dihydroxy-2,3 propyl selon l'invention présente d'excellentes propriétés moussantes, mouillantes, dispersantes, émulsionnantes, solubilisantes et possède, en outre, une activité anti-pelliculaire, la capacité d'adoucir et de raffermir la peau ainsi que la possibilité de former des vésicules pour la stabilisation et le transport de principes actifs. Par conséquent, les composés selon l'invention sont utiles comme agent tensio-actif, base lavante, agent anti-pelliculaire et agent conditionneur du cheveu.

Les composés selon l'invention sont particulièrement recommandés pour préparer des compositions détergentes, notamment destinées au milieu industriel, ou cosmétiques. Ces compositions, qui constituent également un objet de la présente invention, peuvent contenir un seul des composés de formule (I) ou un mélange desdits composés.

Les compositions détergentes selon l'invention sont caractérisées en ce qu'elles contiennent de 0,1 à 60 % et de préférence 10 à 40 % en poids de (oxo-1 alkyl) amino-2, déoxy-2 glucopyranoside de dihydroxy-2,3 propyl et de 40 à 99,9 % et de préférence 60 à 90 % en poids d'une base détergente et/ou d'un adjuvant.

La base détergente est généralement choisie parmi les tensio-actifs anioniques, non ioniques, cationiques ou amphotères, et les mélanges de ces composés.

L'adjuvant est généralement choisi parmi les adjuvants ou les mélanges d'adjuvants connus dans le domaine des lessives liquide ou en poudre, notamment les zéolites et les complexants du calcium et du magnésium.

Les compositions cosmétiques objet de l'invention sont caractérisées en ce qu'elles contiennent de 0,1 à 50 % et de préférence 5 à 35 % en poids de (oxo-1 alkyl) amino-2, déoxy-2 glucopyranoside de dihydroxy-2,3 propyl et de 50 à 99,9 % et de préférence 65 à 95 % en poids d'un excipient et/ou d'une base détergente et/ou d'un adjuvant.

Les compositions cosmétiques peuvent se présenter sous la forme de savon liquide doux, shampooing, bain moussant, gel douche ou formule de soin, notamment pommade, crème et lait de beauté.

Lorsque la composition est un savon liquide doux, elle renferme de 5 à 30 % en poids et de préférence de 5 à 20 %, de (oxo-1 alkyl) amino-2, déoxy-2 glucopyranoside de dihydroxy-2,3 propyl et 70 à 95 % et de préférence 80 à 95 % en poids d'un excipient.

L'excipient est généralement choisi parmi les tensio-actifs anioniques tels que le cocoyl isethionate de sodium, le lauryl sulfate de sodium ou le sel de sodium d'un alkyl peptide, les tensio-actifs amphotères tels que l'alkyl amidopropyl bétaïne, en particulier la cocoamidopropyl bétaïne, les huiles minérales lourdes, les dérivés cellulosiques tels que la carboxyméthylcellulose, les solvants tels que les alcools et plus particulièrement l'éthanol ou le propylène glycol, des complexants tels que l'EDTA, le chlorure de sodium, les alcools gras tels que l'alcool cétylique, des conservateurs, des parfums, des colorants et les mélanges de ces composés.

Lorsque la composition selon l'invention est un shampooing, notamment doux et à usage fréquent, anti-pelliculaire doux, traitant ou à effet conditionneur du cheveu, elle renferme de préférence de 5 à 35 % en poids d'une base détergente contenant de 10 à 75 % en poids de (oxo-1 alkyl) amino-2, déoxy-2 glucopyranoside de dihydroxy-2,3 propyl et de 65 à 95 % en poids d'un adjuvant.

La base détergente est généralement choisie parmi les alkyl éther sulfates tels que le lauryl éther sulfate de sodium ou de magnésium, les alkyl éther sulfates polyoxyéthylénés tels que le lauryl éther sulfate de sodium polyoxyéthyléné, les alkyl bétaïnes telles que la cocoylbétaïne, les alkyl amidopropylbétaïnes telles que la cocoyl amidopropyl bétaïne, une alkyl diméthylamino acétique acide bétaïne telle que la lauryl diméthylamino acétique acide bétaïne, les alkyl diméthylamino hydroxypropyl sulfobétaïne, les α-oléfine sulfonates de sodium, les alkyl polyéthylène glycols tels que l'octadécyl PEG 15, les alkyl imidazolium bétaïnes telles que la cocoyl imidazolium bétaïne, les alkyl éther sulfosuccinates tels que le lauryl éther sulfosuccinate de disodium, les β-alkyl amino propionates tels que le sodium β-laurylamino propionate, les alkyl diamino éthyl glycines telles que le sodium lauryl alkyl diamino éthyl glycine, et les mélanges de ces composés.

L'adjuvant est généralement choisi parmi les épaississants, les texturants tels que les diéthanolamides d'acides gras, plus particulièrement le cocoyl diéthanolamide, et les alkyl acrylates, plus particulièrement le laurylacrylate, le chlorure de sodium, les dialkylcarboxylate d'éthylène glycol, plus particulièrement le distéarate d'éthylène glycol, les oxydes d'amine grasse, plus particulièrement le N-cocoyl oxyde, lesquels composés sont généralement utilisés à raison de 0-10 % en poids.

L'adjuvant peut également être choisi parmi les agents de conditionnement, les adoucissants tels que des hydrolysats de protéines de blé et les éthers de cellulose contenant des ammoniums quaternaires (teneur en azote : 1-3 %, PM : 50-150 000), les copolymères acrylamide/chlorure de diméthyl alkyl ammonium (0,5-5 % en poids), les complexants tels que l'EDTA et l'acide galactarique (0,1-1 % en poids), les parfums, les agents nacrants, les conservateurs, les acidifiants, l'eau et les mélanges de ces composés.

Lorsque la composition selon l'invention est un bain moussant, elle renferme de préférence de 5 à 35 % en poids d'une base détergente contenant plus de 50 % en poids de (oxo-1 alkyl) amino-2, déoxy-2 glucopyranoside de dihydroxy-2,3 propyl et de 65 à 95 % en poids d'un adjuvant.

La base détergente est généralement choisie parmi les composés connus de l'homme du métier. A titre d'exemples, on peut citer les alkyl amido bétaïnes telles de la cocoylamido propyl bétaïne, les alkyl carboxylates de sorbitan éthoxylés tels que le laurate de sorbitan éthoxylé et les mélanges de ces composés.

L'adjuvant est généralement choisi parmi les mono- ou triéthanolamides d'acides gras (0-10 % en poids), les dialkylcarboxylates de propylène glycol éthoxylé (0-5 % en poids), les polyéthylène glycols tels que le triéthylène glycol (0-5 % en poids), les alkyl acryliques tels que l'oléyl acrylique (0-5 % en poids), les huiles végétales telles que l'huile d'amandes douces (0-10 % en poids), le chlorure de sodium, l'EDTA (0-5 % en poids), les alcools gras tels que l'hexadécanol (0-2 % en poids), les conservateurs, les parfums, les colorants, l'eau et les mélanges de ces composés.

Lorsque la composition selon l'invention est un gel douche, elle renferme de préférence de 5 à 35 % en poids d'une base détergente contenant au moins 50 % en poids de (oxo-1 alkyl) amino-2, déoxy-2 glucopyranoside de dihydroxy-2,3 propyl et de 65 à 95 % en poids d'un adjuvant.

La base détergente est généralement choisie parmi les alkyl sulfosuccinates polyoxyéthylénés tels que le cocoyl sulfo succinate à 3 moles d'oxyde d'éthylène, les alkyl amido-N-glycinates, les alkyl sulfates d'ammonium tels que le lauryl sulfate d'ammonium et les mélanges de ces composés.

L'adjuvant est généralement choisi parmi les alkylcarboxylates de propylène glycol éthoxylés tels que le dioléate de propylène glycol éthoxylé (0-5 % en poids), les alkyl amido bétaïnes telle que la lauryl amidopropyl bétaïne (0-5 % en poids), les agents nacrants (0-7 % en poids), les gels acryliques (0-1 % en poids), le chlorure de sodium, les complexants, les conservateurs, les parfums, l'eau purifiée et les mélanges de ces composés.

La composition selon l'invention peut enfin être une formule de soins pour le visage, notamment une pommade, une crème, un lait de beauté, présentant une structure de type gel.

Les exemples qui suivent permettent d'illustrer l'invention sans toutefois la limiter.

Dans ces exemples, on utilise les méthodes de mesure suivantes :

Le pouvoir moussant est mesuré par bullage d'azote à débit constant (1 l/h) à travers un fritté (perméabilité à l'eau : 0,11 l/h) placé à la base d'une éprouvette graduée (32 mm x 210 mm) et thermostatée contenant 20 ml d'une solution aqueuse renfermant 3 g/l de (oxo-1 alkyl) amino-2, déoxy-2 glucopyranoside de dihydroxy-2,3 propyl et 12 g/l de glycérol. La quantité de mousse générée par bullage (1 minute et 40 secondes) est estimée volumétriquement immédiatement après l'arrêt du bullage. Le volume de la mousse ainsi formée est exprimé en ml.

Le pouvoir mouillant est mesuré selon le test qui consiste à suivre durant 600 secondes, la quantité de solution à tester (3 g de matières actives par litre de glycérol; 25°C) absorbée par un tissu de coton écru (2,5 cm x 2 cm; 0,145 g; selon la norme NF T73-406 (décembre 1975). La pièce de tissu effleure la surface de la solution de tensio-actifs et la traction générée par la montée capillaire de la solution est enregistrée en continu (tensiomètre KRUSS automatique muni du logiciel d'absorption K 121. On mesure la masse absorbée par le tissu à saturation (MS; en g) et le temps nécessaire pour atteindre la saturation (TS; en s).

L'abaissement de la tension superficielle de l'eau est mesuré par tensiométrie classique selon la norme ISO 304 modifiée en ce que les dimensions de la platine rectangulaire sont 25 mm x 5 mm x 0,1 mm. La tension superficielle γ est exprimée en mN/m.

Les exemples qui suivent permettent d'illustrer l'invention sans toutefois la limiter.

### EXEMPLES 1 à 6

A 21,57g (0,10 mole) de chlorhydrate de glucosamine (G, 220-6; ALDRICH) solubilisés dans 200 ml d'une solution aqueuse de soude (0,5 N) à 20°C, on ajoute, à un débit de 1 ml/min et sous agitation (100 tours/min; axe à double pâle d'agitation), 20, 3 g de chlorure d'acide octanoïque (exemple 1; ALDRICH Réf. 0,473-3), décanoïque (exemple 2; ALDRICH Réf. 14,029-5), undécanoïque (exemple 3; ALDRICH Réf. 24,943-2), undécylénique (exemple 4; ALDRICH Réf. 16, 166-7), dodécanoïque (exemple 5; ALDRICH Réf. 15, 693-0) ou tétradécanoïque (exemple 6; ALDRICH Réf. 29, 861-1) et, simultanément, une solution de soude 4 N afin de fixer et maintenir le pH à 9,5. Après 2 heures d'agitation complémentaire, on ajuste le pH à 6 par ajout d'une solution aqueuse d'acide méthane sulfonique à 70 % en poids. Il se forme un précipité qui est filtré, essoré, lavé deux fois avec 60 ml d'eau et à nouveau essoré sur filtre en verre fritté conventionnel. Le précipité ainsi obtenu est un mélange des anomères α et β du (oxo-1 alkyl) amino-2, déoxy-2 glucopyranose correspondant dont le rendement rapporté à la matière sèche est indiqué dans le tableau 1.

10 g du précipité obtenu précédemment sont mis en présence de 51,3 g (0,557 mole) de glycérol (13, 487-2; ALDRICH) et 0,48 g (5 x 10⁻³ mole) d'acide méthane sulfonique sous agitation (300 tours/min.) pendant 4 heures, à 110°C et sous un vide de 30 mm de Hg.

Le milieu réactionnel limpide ainsi obtenu est refroidi à 40°C, dilué avec 500 ml d'éthanol absolu et le pH est ajusté à 6 par addition d'hydrogénocarbonate de sodium en poudre. Le mélange obtenu est filtré (verre fritté) et le filtrat est concentré dans un évaporateur rotatif (vide : 2,5 KPa; température : 60°C). On récupère ainsi une huile qui contient les anomères α et β du (oxo-1 alkyl) amino-2, déoxy-2 glucopyranoside de dihydroxy-2,3 propyl correspondant (20 % en poids dans le glycérol).

L'huile obtenue est analysée par chromatographie sur couche mince (CCM) sur une plaque de silice greffée (RP18; MERCK) éluée avec un mélange MeOH/H₂O 75:25 v:v. Les spots de migration sont révélés à l'aide d'une solution d'acide sulfurique à 50 % dans l'eau et chauffage à 120°C pendant 2 minutes. On observe un spot de forme allongée dont la valeur du Rf est indiquée dans le tableau 1.

Les caractéristiques physico-chimiques de l'huile précitée sont présentées dans le tableau 1.

**TABLEAU 1**

| **Exemple** | **Rendement %** | **Rf** | **Pouvoir moussant (ml)** | **Pouvoir mouillant** | | **Tension superficielle γ (mN/m)** |
|---|---|---|---|---|---|---|
| | | | | **MS (g)** | **TS (s)** | |
| 1 | 64,0 | 0,72 | 8 | 0,185 | 900 | 27,2 |
| 2 | 95,6 | 0,44 | 120 | 0,263 | 150 | 27,4 |
| 3 | 92,0 | 0,35 | 78 | 0,265 | 400 | 30,5 |
| 4 | 90,0 | 0,60 | 130 | 0,250 | 70 | 27,5 |
| 5 | 85,0 | 0,25 | 100 | 0,253 | 230 | 30,3 |
| 6 | 88,2 | 0,14 | 50 | 0,248 | 700 | 29,7 |
| Glycérol | - | - | 0 | - | - | - |
| APG* | - | - | 100 | - | - | - |

| | | | | | | |
|---|---|---|---|---|---|---|
| * alkylpolyglucoside (HENKEL Ref. APG 1200) | | | | | | |

On constate que le pouvoir moussant des composés des exemples 4, 2 et 5 est supérieur ou égal à celui de l'APG, produit de référence en la matière.

On observe que la mousse formée lors de la mesure du pouvoir moussant est stable et que la décroissance du volume de la mousse est inférieure à 20 % après 20 minutes (exemples 1 à 6).

Les anomères α et β de l'huile selon l'exemple 4 sont séparés par chromatographie-flash (colonne silice 35-70 µm, longueur : 25 cm; diamètre : 5,6 cm; éluant : CHCl₃, puis CHCl₃/MeOH, détection réfractométrique.

Les anomères α et β ainsi obtenus sont caractérisés par les méthodes analytiques suivantes :
- chromatographie sur couche mince : Rf identique égal à 0,6 pour chaque anomère (plaque de silice; épaisseur : 200 µm; taille des particules : 5-10 µm; éluant CHCl₃/MeOH 8:2 v:v).
- chromatographie liquide haute performance (HPLC) : temps de rétention (T_{R}) égal à 17,4 min. et 14,7 min. respectivement (colonne LICHROSPHER RP18; diamètre : 4 mm; longueur : 125 mm; taille des particules : 5 µm; éluant : MeOH/H₂O 50:50 v:v; débit : 1 ml/min.; détection : U.V. (λ : 220 nm) et réfractométrie différentielle.
- spectroscopie I.R. (pastille KBr 1 %) : νOH : 3350 cm⁻¹; δCH2 : 2853 et 2825 cm⁻¹; νCO-NH2 : 1642 et 1552 cm⁻¹.
- RMN ¹³C 200 MHz; pyridine d₅ :

| Anomère α | |
|---|---|
| Carbone | Déplacement chimique (ppm) |
| C1 | 99,22 - 99,02 |
| C2 | 55,48 |
| C3 | 71,71 |
| C4 | 74,31 |
| C5 | 82,31 |
| C6 | 70,55 |
| (CH₂)ₙ | 36,59 - 33,95 - 29,58 - 29,26 - 29,04 - 26,24 |
| CO | 174,40 |
| C1' | 62,59 - 62,04 |
| C2' | 72,89 - 72,67 |
| C3' | 64,62 - 64,44 |
| -CH=CH₂ | 139,32 |
| -CH=CH₂ | 114,52 |

| Anomère β: | |
|---|---|
| Carbone | Déplacement chimique (ppm) |
| CO | 174,84 |
| C1 | 103,04 |
| C2 | 57,38 |

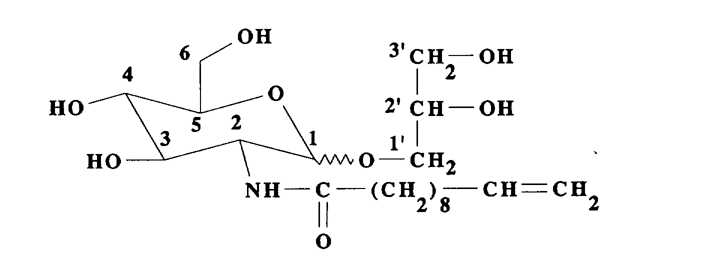

## Revendications

1. Composé de formule : 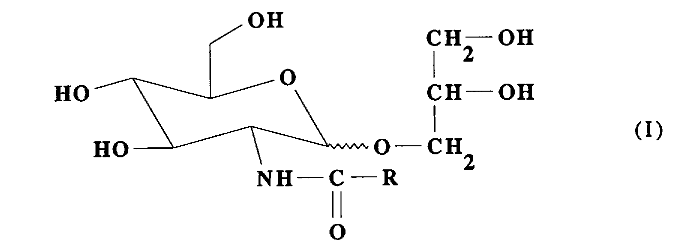 dans laquelle R représente un groupement alkyle, linéaire ou ramifié, saturé ou insaturé, renfermant de 5 à 21 atomes de carbone.

2. Composé selon la revendication 1 caractérisé en ce que le radical R renferme de 7 à 17 atomes de carbone.

3. Procédé de préparation du composé selon l'une des revendications 1 ou 2 caractérisé en ce qu'il consiste à
a) former une N-acyl glucosamine à partir de chlorhydrate de glucosamine et de chlorure d'acide en C₆-C₂₂, et
b) faire réagir le composé de l'étape a) avec le glycérol en présence d'un catalyseur acide.

4. Procédé selon la revendication 3 caractérisé en ce que le rapport molaire chlorure d'acide/chlorhydrate de glucosamine est compris entre 0,7 et 1,3.

5. Procédé selon l'une des revendications 3 ou 4 caractérisé en ce que le rapport du nombre d'équivalents en mole de glycérol au nombre d'équivalents en moles de N-acyl glucosamine de l'étape b) est compris entre 1 et 50.

6. Procédé selon l'une des revendications 3 à 5 caractérisé en ce que le catalyseur est choisi parmi le groupe constitué par les acides, les résines sous forme H⁺ et les argiles acides.

7. Procédé selon la revendication 6 caractérisé en ce que le rapport du nombre d'équivalents en mole de l'acide au nombre d'équivalents en mole de N-acyl glucosamine est compris entre 10⁻³ et 1.

8. Procédé selon la revendication 6 caractérisé en ce que la quantité de résine ou d'argile exprimée en équivalent pondéral calculé sur la base de 1 équivalent en mole de N-acyl glucosamine est comprise entre 0,05 et 6.

9. Composition détergente caractérisée en ce qu'elle comprend de 0,1 à 60 % en poids du composé selon l'une des revendications 1 ou 2, et de 40 à 99,9 % en poids d'une base détergente et/ou d'un adjuvant.

10. Composition cosmétique caractérisée en ce qu'elle comprend de 0,1 à 50 % en poids du composé selon l'une des revendications 1 ou 2, et de 50 à 99,9 % en poids d'un excipient et/ou d'une base détergente et/ou d'un adjuvant.

11. Composition selon la revendication 10 caractérisée en ce quelle appartient au groupe constitué par les savons liquides doux, les shampooings, les bains moussants, les gels douche et les formules de soin, notamment les pommades, crèmes et laits de beauté.

12. Utilisation du composé selon l'une des revendications 1 ou 2 comme agent tensio-actif.

13. Utilisation du composé selon l'une des revendications 1 ou 2 comme base lavante.

14. Utilisation du composé selon l'une des revendications 1 ou 2 comme agent anti-pelliculaire.

15. Utilisation du composé selon l'une des revendications 1 ou 2 comme agent conditionneur du cheveu.

## Patentansprüche

1. Verbindung der Formel: 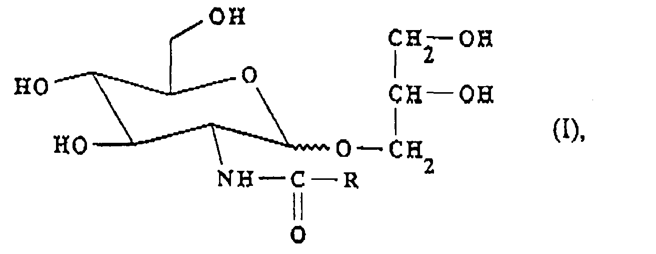 worin R eine geradkettige oder verzweigte, gesättigte oder ungesättigte Alkylgruppe mit 5 bis 21 Kohlenstoffatomen bedeutet.

2. Verbindung nach Anspruch 1, dadurch gekennzeichnet, daß die Gruppe R 7 bis 17 Kohlenstoffatome enthält.

3. Verfahren zur Herstellung einer Verbindung nach einem der Ansprüche 1 oder 2, dadurch gekennzeichnet, daß es darin besteht,
a) aus einem Glucosamin-Hydrochlorid und dem Chlorid einer C₆₋₂₂-Säure ein N-Acyl-Glucosamin zu bilden, und
b) die Verbindung aus Schritt a) mit Glycerin in Gegenwart eines Säurekatalysators umzusetzen.

4. Verfahren nach Anspruch 3, dadurch gekennzeichnet, daß das Molverhältnis Säurechlorid/Glucosamin-Hydrochlorid im Bereich von 0,7 bis 1,3 liegt.

5. Verfahren nach einem der Ansprüche 3 oder 4, dadurch gekennzeichnet, daß das Verhältnis Moläquivalente Glycerin / Moläquivalente N-Acylglucosamin in Schritt b) im Bereich von 1 bis 50 liegt.

6. Verfahren nach einem der Ansprüche 3 bis 5, dadurch gekennzeichnet, daß der Katalysator unter den Säuren, Harzen in H⁺-Form und sauren Tonen ausgewählt ist.

7. Verfahren nach Anspruch 6, dadurch gekennzeichnet, daß das Verhältnis Moläquivalente Säure / Moläquivalente N-Acylglucosamin im Bereich von 10⁻³-bis 1 liegt.

8. Verfahren nach Anspruch 6, dadurch gekennzeichnet, daß der Mengenanteil der Harze oder Tone in Gewichtsäquivalenten, auf der Basis von 1 Moläquivalent N-Acylglucosamin berechnet, im Bereich von 0,05 bis 6 liegt.

9. Reinigende Zusammensetzung, dadurch gekennzeichnet, daß sie 0,1 bis 60 Gew.-% einer Verbindung nach einem der Ansprüche 1 oder 2 und 40 bis 99,9 Gew.-% reinigende Basisformulierung und/oder Zusatzstoff enthält.

10. Kosmetische Zusammensetzung, dadurch gekennzeichnet, daß sie 0,1 bis 50 Gew.-% einer Verbindung nach einem der Ansprüche 1 oder 2 und 50 bis 99,9 Gew.-% Grundmasse und/oder reinigende Basisformulierung und/oder Zusatzstoff enthält.

11. Zusammensetzung nach Anspruch 10, dadurch gekennzeichnet, daß sie unter den milden flüssigen Seifen, Haarwaschmitteln, Schaumbädern, Duschgelen und Formulierungen zur Pflege, insbesondere Pomaden, Cremes und Schönheitsmilchen, ausgewählt ist.

12. Verwendung der Verbindung nach einem der Ansprüche 1 oder 2 als grenzflächenaktiven Stoff.

13. Verwendung der Verbindung nach einem der Ansprüche 1 oder 2 als reinigende Basisformulierung.

14. Verwendung der Verbindung nach einem der Ansprüche 1 oder 2 als Mittel gegen Schuppen.

15. Verwendung der Verbindung nach einem der Ansprüche 1 oder 2 als Konditioniermittel für das Haar.

## Claims

1. Compound of formula : 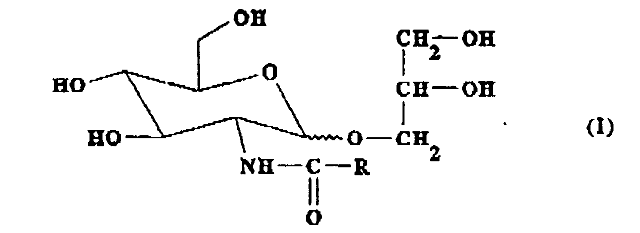 in which R denotes a saturated or unsaturated, linear or branched alkyl group containing from 5 to 21 carbon atoms.

2. Compound according to Claim 1, characterized in that the radical R contains from 7 to 17 carbon atoms.

3. Process for the preparation of the compound according to either of Claims 1 and 2, characterized in that it consists in
a) forming an N-acylglucosamine from glucosamine hydrochloride and C₆-C₂₂ acid chloride, and
b) reacting the compound from stage a) with glycerol in the presence of an acid catalyst.

4. Process according to Claim 3, characterized in that the acid chloride/glucosamine hydrochloride molar ratio is between 0.7 and 1.3.

5. Process according to either of Claims 3 and 4, characterized in that the ratio of the number of molar equivalents of glycerol to the number of molar equivalents of N-acylglucosamine from stage b) is between 1 and 50.

6. Process according to one of Claims 3 to 5, characterized in that the catalyst is chosen from the group consisting of acids, resins in H⁺ form and acidic clays.

7. Process according to Claim 6, characterized in that the ratio of the number of molar equivalents of the acid to the number of molar equivalents of N-acylglucosamine is between 10⁻³ and 1.

8. Process according to Claim 6, characterized in that the quantity of resin or of clay, expressed as weight equivalent calculated on the basis of 1 molar equivalent of N-acylglucosamine, is between 0.05 and 6.

9. Detergent composition characterized in that it includes from 0.1 to 60 % by weight of the compound according to either of Claims 1 and 2, and from 40 to 99.9 % by weight of a detergent base and/or of an adjuvant.

10. Cosmetic composition characterized in that it includes from 0.1 to 50 % by weight of the compound according to either of Claims 1 and 2, and from 50 to 99.9 % by weight of an excipient and/or of a detergent base and/or of an adjuvant.

11. Composition according to Claim 10, characterised in that it belongs to the group consisting of mild liquid soaps, shampoos, foam baths, shower gels and care formulations, especially ointments, creams and beauty milks.

12. Use of the compound according to either of Claims 1 and 2 as surface-active agent.

13. Use of the compound according to either of Claims 1 and 2 as waahing base.

14. Use of the compound according to either of Claims 1 and 2 as antidandruff agent.

15. Use of the compound according to either of Claims 1 and 2 as hair-conditioning agent.
